Europäisches Patentamt

European Patent Office

Office européen des brevets

(11) Numéro de publication: **0 404 607**

**A2**

# (12) DEMANDE DE BREVET EUROPEEN

(21) Numéro de dépôt: **90401292.9**

(22) Date de dépôt: **16.05.90**

(51) Int. Cl.5: **C07C 319/00, C10M 135/24, C07C 319/22, C10M 135/30, C10M 135/32**

(30) Priorité: **18.05.89 FR 8906507**

(43) Date de publication de la demande:
**27.12.90 Bulletin 90/52**

(84) Etats contractants désignés:
**BE DE ES FR GB IT NL**

(71) Demandeur: **ELF FRANCE**
**Tour Elf 2, Place de la Coupole**
**LA DEFENSE 6 - 92400 Courbevoie(FR)**

(72) Inventeur: **Germanaud, Laurent**
**Route de Lafayette, Valancin le Fayet**
**F-38540 Heyriaux(FR)**
Inventeur: **Azorin, Patrick**
**Lotissement Joly, La Plaine**
**F-69440 Mornant(FR)**
Inventeur: **Lhopital, Michel**
**Le Grillon - Bât. A**
**F-69340 Francheville(FR)**

(74) Mandataire: **Boillot, Marc**
**SOCIETE NATIONALE ELF AQUITAINE**
**Division Propriété Industrielle Tour Elf**
**F-92078 Paris la Défense Cédex 45(FR)**

(54) **Additifs sulfures aux lubrifiants à effet anti-usure et extrême pression, leurs procédés de préparation et compositions renfermant lesdits additifs.**

(57) Cette invention concerne des additifs aux lubrifiants à effet antiusure et extrême-pression consistant en dihydrocarbylpolysulfures asymétriques renfermant un groupement hydrocarbylpolysulfure $R-S_x-$ en position vicinale d'un alcool secondaire ou tertiaire, ainsi que des compositions lubrifiantes renfermant ces additifs. Les additifs sont préparés par réaction d'un sel basique d'un hydrocarbyl(poly)sulfure soit avec un composé renfermant au moins un groupement époxydique, soit avec un thiosulfate organique alpha-hydroxylé.

EP 0 404 607 A2

# ADDITIFS SULFURES AUX LUBRIFIANTS A EFFET ANTIUSURE ET EXTREME PRESSION, LEURS PROCEDES DE PREPARATION ET COMPOSITIONS RENFERMANT LESDITS ADDITIFS

La présente invention concerne des composés organiques sulfurés utilisables comme additifs à effet antiusure et extrême-pression aux lubrifiants, la synthèse de ces composés et des compositions lubrifiantes renfermant lesdits composés.

Les additifs extrême-pression sont utilisés principalement dans les huiles pour engrenages industriels ou automobiles ou pour les lubrifiants spécifiques à l'usinage des métaux. Ces composés en général sulfurés, chlorés, phosphorés ou azotés, agissent en formant une couche superficielle qui empêche la formation de microsoudures locales. Ces microsoudures sont générées par des effets thermiques de grand amplitude apparaissant entre deux surfaces en mouvement l'une par rapport à l'autre sous des contraintes élevées.

Les additifs extrême pression actuellement utilisés peuvent être classés en quatre groupes.

- Le premier groupe concerne les produits résultant de la réaction des alcools, des phénols, des oléfines et/ou des composés hydrocarbonés contenant des atomes de soufre, de chlore et d'azote avec des dérivés du phosphore tel que le pentasulfure de phosphore. Ces produits sont décrits par exemple dans le brevet américain US 4 058 468. L'effet extrême pression de ces additifs soufrés et phosphorés est généralement insuffisant mais ils présentent des propriétés supplémentaires intéressantes. En particulier ils sont antioxydants et non corrosifs.

- Le deuxième groupe intéresse les produits formés par réaction des oléfines mono ou polyinsaturées ou des composés aromatiques éventuellement substitués par des chaines alkyle, ou des chaines hydrocarbonées contenant des hétéroatomes comme le soufre, le chlore, le phosphore ou l'azote, ou encore des esters d'acides gras avec les composés chlorés du soufre ($SCl_2$, $S_2Cl_2$, R-S-Cl).

Le produit obtenu est traité dans certains cas par un hydroxyde alcalin, un mercaptide de sodium ou un polysulfure de sodium pour réduire la teneur en chlore résiduel. Ces produits sont décrits par exemple dans les brevets américains US 4 204 269, US 4 198 305, US 4 097 387, US 3 925 414, US 3 844 964 et le brevet français BF 2 404 042. Le brevet français BF 2 588 881 recommande d'ajouter un chlorure d'alkényle avant le traitement par un polysulfure alcalin pour améliorer la solubilité des produits finaux dans l'huile.

Le brevet français FR 2 605 328 préconise le traitement ultérieur par un polysulfure organique en remplacement du polysulfure de sodium pour améliorer la solubilité des produits finaux dans les huiles minérales et synthétiques.

Les produits obtenus selon ces procédés ont des teneurs en soufre moyennes à élevées (jusqu'à 50 %) leur conférant des propriétés extrême pression excellentes, mais ils sont souvent corrosifs. Un des inconvénients majeurs dans la synthèse de ces produits concerne les étapes finales de lavage qui augmentent le coût du procédé. Ces produits contiennent généralement un peu de chlore résiduel ce qui peut limiter leur emploi dans certaines utilisations.

- Le troisième groupe comprend les produits issus de la réaction des oléfines mono ou polyinsaturées, des esters d'acides gras insaturés ou des composés hydrocarbonés contenant des hétéroatomes comme le soufre, le chlore, le phosphore ou l'azote avec du soufre élémentaire ou un mélange de soufre élémentaire et d'hydrogène sulfuré, en présence ou en absence de catalyseur. On connaît par exemple les procédés mettant en oeuvre la réaction de l'isobutylène ou des oléfines en $C_3$ à $C_8$ avec du soufre comme le mentionnent les brevets US 4 119 850, US 4 119 545 et US 3 899 475.

- Le quatrième groupe comprend les produits d'addition des dérivés minéraux du soufre aux époxydes.

Le brevet allemand DE 36 04793 revendique la préparation d'additifs antiusure et extrême pression par condensation d'époxydes obtenus préférentiellement par époxydation d'alpha-oléfines de 6 à 20 atomes de carbone avec un polysulfure de sodium comme $Na_2S_2$ ou $Na_2S_4$. Les produits contiennent en général de 15 à 30 % de soufre et sont non corrosifs vis-à-vis des métaux nobles.

Le brevet US 3 064 056 indique la préparation de bis-(béta-hydroxyalkyl) di ou trisulfures par réaction d'un époxyde avec du soufre élémentaire en présence de catalyseur basique.

Tous ces produits ont une structure symétrique, ce qui limite considérablement le nombre de variantes possibles.

Nous avons trouvé maintenant une nouvelle famille d'additifs sulfurés de structure asymétrique. Cette structure asymétrique permet d'ajuster beaucoup plus finement aussi bien la solubilité de ces produits dans l'huile que leurs performances antiusure et extrême-pression.

Pour cela, les nouveaux additifs sulfurés aux lubrifiants à effet antiusure, et extrême-pression sont caractérisés en ce qu'ils consistent en dihydrocarbylpolysulfures asymétriques renfermant un groupement

hydrocarbylpolysulfure $R-S_x-$ en position vicinale d'un alcool secondaire ou tertiaire.

R représente un radical aliphatique en général en $C_1$ à $C_{18}$ et de préférence en $C_1$ à $C_{14}$, pouvant comporter un ou plusieurs hétéroatomes tel que l'oxygène, le soufre, le phosphore ou l'azote, un radical aromatique, éventuellement substitué par un ou plusieurs radicaux aliphatiques pouvant renfermer des hétéroatomes ou un radical hétérocyclique renfermant au moins un hétéroatome choisi parmi le soufre, l'oxygène au l'azote.

La valeur de x peut varier entre 2 et 10 et de préférence entre 2 et 6.

Les groupements $R-S_x-$ proviennent de mercaptans aliphatiques, aromatiques ou hétérocycliques correspondants.

Les atomes de soufre supplémentaires sont introduits à l'aide de dérivés minéraux du soufre, comme le soufre élémentaire ou les thiosulfates alcalins comme le thiosulfate de sodium ou d'ammonium.

Selon le mode de réalisation directe de l'invention on prépare un sel basique d'un polysulfure organique $R-S_xM$ par réaction du sel basique d'un mercaptan, R-SM avec au moins un équivalent de soufre élémentaire, puis on fait réagir ce polysulfure sur un époxyde.

Dans la formule R-SM, R a la signification précitée, tandis que M représente un métal alcalin ou alcalinoterreux ou encore un groupement monovalent, comme $NH_4^+$, correspondant à une base minérale MOH.

Un autre procédé consiste à préparer d'abord un thiosulfate organique alpha-hydroxylé par addition d'un thiosulfate alcalin sur un composé renfermant au moins un groupement époxydique. Le thiosulfate organique alpha-hydroxylé réagit dans un deuxième temps avec le sel basique d'un mercaptan R-SM ou d'un polysulfure organique $R-S_xM$ où R,M et x ont la signification précitée.

Parmi les mercaptans aliphatiques utilisables pour préparer les produits de l'invention on peut citer le méthylmercaptan, l'éthylmercaptan, le propylmercaptan, le n-butylmercaptan, l'isobutylmercaptan, le terbutylmercaptan, le teramylmercaptan, le ternonylmercaptan, le terdodécylmercaptan et le benzylmercaptan. Parmi les mercaptans aliphatiques comportant un ou plusieurs hétéroatomes nous citons le mercaptoéthanol, le mercapto-3-propanediol-1,2, l'acide mercapto-acétique ou thioglycolique et l'acide mercapto-propionique.

Parmi les mercaptans aromatiques, nous mentionnons le phénylmercaptan ou thiophénol, les tolylmercaptans et l'acide ortho-thiobenzoïque ou acide thiosalicylique.

Parmi les mercaptans hétérocycliques nous mentionnons le mercapto-2-imidazole, le mercapto-2-méthylimidazole, le mercapto-2-benzimidazole, le mercapto-2-benzoxazole, le mercapto-2-pyridine, le mercapto-4-pyridine, le mercapto-2-thiazole, le mercapto-2-thiazole, le mercapto-2-méthyl-5-thiadiazole-1,3,4 et le mercapto-3-méthyl-4-triazole-1,2,4.

Les composés renfermant au moins un groupement époxydique ont une structure hydrocarbonée acyclique ou cyclique.

Les groupements époxydiques peuvent être indifféremment en position terminale ou en milieu des structures acycliques. En effet, il est connu que l'ouverture des époxydes en position terminale par un mercaptan ou un alkylpolysulfure conduit à des alcools secondaires ou tertiaires. Seul, l'oxyde d'éthylène, dont l'ouverture dans ces conditions donne un alcool primaire, est exclu du champ de l'invention.

Parmi les composés acycliques ayant au moins un groupement époxydique en position terminale on peut citer l'époxy-1,2-propane, l'époxy-1,2-butane, l'époxy-1,2-héxane, l'époxy-1,2,-octane, l'époxy-1,2-dodécane et l'époxy styrène ou les diépoxydes, comme le diépoxy-1,2, 3,4-butane.

Parmi les composés acycliques ayant des groupements époxydiques en milieu de chaîne on peut citer les époxydes d'acides gras insaturés, tels que l'acide oléique ou l'oléate de méthyle époxydés, le linoléate de méthyle époxydé ou l'huile de soja époxydée.

Parmi les époxydes cycliques on peut mentionner l'époxy-1,2-cyclohéxane.

Il peut être avantageux d'utiliser des composés qui renferment un groupe fonctionnel comme un halogène, un alcool ou un éther en position vicinale par rapport à l'époxyde. On peut citer l'épichlorhydrine, l'épibromhydrine et les éthers alkylglycidiques comme l'éther n-butylglycidique, l'éther éthyl-2-héxylglycidique ou l'éther phénylglycidique.

Les sels basiques des mercaptans peuvent être obtenus par réaction, de préférence en milieu alcoolique, d'un mercaptan avec une base minérale MOH. On utilise le plus couramment la soude, la potasse ou l'ammoniaque. Le milieu alcoolique peut comprendre au moins un monoalcool aliphatique, comme le méthanol, l'éthanol ou l'isopropanol. La réaction est en général effectuée à une température de 20 à 100°C.

Les sels basiques des mercaptans peuvent être transformés en polysulfures par réaction avec un ou plusieurs équivalents de soufre élémentaire.

Selon le mode de réalisation directe, les polysulfures sous forme de leurs sels basiques réagissent

3

avec l'époxyde en milieu alcoolique ou hydroalcoolique, à une température comprise entre environ 20 et 120°C et de préférence entre 50 et 70°C. Parmi les alcools on utilise de préférence le méthanol, l'éthanol ou l'isopropanol. La durée de la réaction est généralement comprise entre 0,5 et 5 heures. Une durée de une heure environ est généralement suffisante.

Les produits sont extraits en fin de réaction par un solvant organique, tel que le toluène ou les xylènes et isolés après évaporation du solvant.

Selon l'autre procédé, on prépare d'abord un thiosulfate organique alpha-hydroxylé par réaction d'un thiosulfate alcalin avec un époxyde.

On utilise en général un mélange de thiosulfate de sodium et de thiosulfate d'ammonium dans un milieu alcoolique ou hydroalcoolique. La durée de la réaction varie entre environ 1 et 5 heures, et la température entre 20 et 100°C. La pâte obtenue après l'évaporation des solvants est purifiée par redissolution et réévaporation.

Le thiosulfate organique alpha-hydroxylé réagit dans un deuxième temps avec le sel basique du mercaptan ou avec le sel basique du polysulfure organique, dans un milieu alcoolique ou hydroalcoolique, à une température comprise entre 20 et 120°C, de préférence entre 50 et 70°C. On utilise de préférence le méthanol, l'éthanol ou l'isopropanol. La durée de la réaction est comprise entre 0,5 et 5 heures, une durée d'une heure étant généralement suffisante. Les produits sont extraits en fin de réaction par un solvant organique, tel que le toluène, les xylènes.

Par réaction avec les mercaptans on introduit des groupements $R-S_x-$ où $x = 2$. L'utilisation des polysulfures correspondants permet d'augmenter le nombre d'atomes de soufre à la valeur désirée.

Les composés sulfurés organiques selon l'invention sont utilisés comme additifs à effet antiusure et extrême-pression pour lubrifiants.

En variant la structure des mercaptans et époxydes utilisés ainsi que le nombre d'atomes de soufre introduits dans la molécule, il est possible d'ajuster la solubilité dans les huiles et l'effet antiusure et extrême-pression. Dans la plupart des cas, la solubilité est totale.

Une première application des additifs de l'invention concerne plus particulièrement la formulation d'huiles destinées à la lubrification des engrenages. Les huiles de base peuvent être d'origine minérale ou synthétique.

Les huiles synthétiques incluent notamment les oligomères d'oléfines tels que les tri-,tétra- et pentamères du décène-1 obtenus par oligomérisation en présence d'acides de LEWIS. D'autres alpha-oléfines peuvent bien sûr être employées, par exemple les alpha-oléfines en $C_6$ à $C_{14}$.

On peut encore utiliser des alkylbenzènes, tels que les mono et dialkylbenzènes, ou encore les esters synthétiques provenant d'acides mono ou polycarboxyliques (tels que l'acide sébacique, les acides gras, etc.) et de monoalcools ou de polyols, (tels que l'éthyl-2-héxanol, le triméthylol-propane, etc.).

Les additifs selon l'invention peuvent être ajoutés aux huiles lubrifiantes à des concentrations allant par exemple de 0,5 à 10 % en masse.

Ces additifs peuvent être utilisés en combinaison avec des additifs phosphorés, tels que les dialkyl ou diaryldithiophosphates métalliques, les phosphites et les phosphates organiques.

D'autres additifs classiques peuvent être ajoutés, tels que des antioxydants, des antirouilles, des passivateurs de cuivre, des antimousse, des réducteurs de frottement, dans des proportions usuelles.

Une seconde application comme additifs extrême-pression pour lubrifiants concerne plus particulièrement la formulation d'huiles destinées au travail des métaux (coupe, formage, etc.).

Dans cette application, la concentration d'additif utilisée est en général de 0,1 à 20 % et de préférence de 0,5 à 5 % en masse par rapport à l'huile lubrifiante. Dans cette application, d'autres additifs classiques peuvent être ajoutés, tels que des paraffines chlorées en une proportion correspondant par exemple à 2-10 % en masse de chlore par rapport à l'huile lubrifiante.

Les exemples suivant illustrent l'invention ; ils ne doivent en aucune manière être considérés comme limitatifs.

EXEMPLE I
———————

a) Dans un erlen de 1000ml, on introduit sous agitation et à température ambiante 300ml d'eau, 125g (0,5 mole) de thiosulfate de sodium, et 75g (0,5 mole) de thiosulfate d'ammonium. Quand l'ensemble est devenu homogène, on ajoute 72g (1 mole) d'époxy-1,2 butane et 250ml d'éthanol. Après une heure de chauffage à reflux, la solution biphasique devient homogène et limpide. Le chauffage est maintenu pendant environ 2h.30min (correspondant au temps au delà duquel on n'observe plus de dégagement d'ammoniac). Après évaporation des solvants, la pâte obtenue est reprise avec de l'éthanol et on réévapore ensuite

4

l'alcool. On obtient une poudre blanche (202g) que l'on caractérise par analyse élémentaire.

C 22,91 (théorique 23,1),H 4,44 (théorique 4,30),Na 11,3 (théorique 11,06)

b) Dans un réacteur de 250ml, on introduit 74ml d'éthanol à 95 %, 4,4g de soude en pastilles, et 9g (0,1 mole) de terbutanethiol. L'ensemble est porté sous agitation à 40°C pendant 30min. Par l'intermédiaire d'une ampoule de coulée on rajoute 60ml d'une solution aqueuse contenant 23g de l'alpha-hydroxy-s-butylthiosulfate préparé en 1a, puis la solution est portée à reflux pendant une heure.

Le produit est ensuite extrait par 3 x 30ml de toluène. Après séchage de la phase organique sur sulfate de magnésium puis évaporation du solvant on récupère 18g d'huile inodore de couleur jaune, contenant 32 % de soufre.

## EXEMPLE II

Dans un réacteur de 250ml, on introduit 50ml de méthanol et 4,8g de soude en pastilles; le mélange est chauffé jusqu'à 60°C environ, jusqu'à dissolution de la soude. On ajoute ensuite goutte à goutte 10,8g de terbutylmercaptan en maintenant la température à 60°C pendant 30 minutes après la fin de l'addition.

·On introduit ensuite 8,7g d'époxy-1,2,-butane (on observe une élévation de la température de 10°C). La solution est maintenue au reflux pendant 30min environ puis on ajoute 200ml de toluène. La phase organique extraite est lavée à l'eau puis séchée sur sulfate de magnésium..Après filtration et évaporation du solvant on récupère 18g d'une huile inodore et incolore. La teneur en soufre est égale à 33,5 % (théorique 33 %).

## EXEMPLE III

Dans un réacteur de 250ml, on introduite 50ml de méthanol et 4g de soude en pastilles; le mélange est chauffé à 50°C jusqu'à dissolution de la soude. On ajoute ensuite goutte à goutte 9g de terbutanethiol en maintenant la température à 60°C pendant 30 minutes environ après la fin de l'addition, puis on introduit dans cette solution homogène 6,4g de soufre en fleur. La température est maintenue pendant 30 minutes supplémentaires. On rajoute ensuite goutte à goutte 7,3g d'époxy-1,2-butane (la température augmente de 10°C). Le chauffage est poursuivi pendant 30 minutes. On rajoute ensuite 150ml de toluène. La phase organique extraite est lavée avec 2x50ml d'eau, puis séchée sur sulfate de magnésium. Après filtration et évaporation su solvant on récupère 20g d'une huile contenant 42,5 % de soufre (théorique 42 %).

## EXEMPLE IV

On opère comme dans l'exemple précédent mais en remplaçant la soude par 10ml d'une solution d'ammoniaque à 20 %. On obtient 17,3g d'une huile contenant 52 % de soufre.

## EXEMPLE V

Dans un réacteur contenant 100g d'éthanol à 95%, on introduit après dissolution de 2,1g (0,05 mole) de soude, 11g (0,05 mole) de terdodécylmercaptan. Le mélange est porté à reflux jusqu'à ce qu'il devienne homogène, puis refroidi à température ambiante avant d'ajouter 11,6g du thiosulfate organique préparé selon l'exemple Ia. La solution est chauffée à reflux pendant une heure. Après refroidissement on rajoute 200ml d'eau pour dissoudre le sulfite de sodium. La phase organique est décantée et la phase aqueuse est extraite avec 2 x 30ml de toluène et 30ml d'éther éthylique. Les phases organiques sont réunies et séchées sur sulfate de magnésium. Après évaporation des solvants on récupère 15,5g d'une huile (théorie 16,5g) contenant 22% de soufre.

## EXEMPLE VI

On opère comme dans l'exemple II en utilisant 11g (0,05 mole) de terdodécylmercaptan, 21g de soude, 1,6g de soufre en fleur et 3,6g d'oxyde de butène.

On récupère 15g d'une huile contenant 21,2% de soufre.

## EXEMPLE VII

On opère comme dans l'exemple I mais en remplaçant le terbutanethiol par le n-butane thiol. On obtient avec un rendement de 75 % une huile contenant 35 % de soufre (théorique 32 %).

## EXEMPLE VIII

Dans cet exemple on évalue les propriétés extrême-pression des produits de l'invention à partir d'une huile minérale 350 Neutral Solvent contenant 1 % des additifs de l'invention, au moyen d'une machine 4 billes selon la procédure ASTM D 2783.

| Additif (1% dans 350N) | charge de grippage | charge de soudure | indice de charge-usure |
|---|---|---|---|
| base seule | 60 | 126 | 22 |
| Ib | 80 | 250 | 32 |
| II | 80 | 250 | 33.7 |
| III | 126 | 315 | 50.1 |
| IV | 100 | 315 | 49.5 |
| V | 100 | 250 | 32 |
| VI | 100 | 250 | 35 |
| VII | 100 | 250 | 33.7 |

Ces résultat montrent que les additifs selon l'invention conduisent à une augmentation sensible de l'indice charge-usure, des charges de grippage et de soudure. Compte tenu de ces performances, ces additifs peuvent être avantageusement utilisés pour la formulation d'huile extrême-pression pour engrenages industriels ou d'automobiles.

## EXEMPLE IX

Dans cet exemple, on évalue l'effet corrosif vis-à-vis du cuivre, des additifs de l'invention, à partir d'essais de corrosion sur lame de cuivre de solutions d'huile minérale 350 Neutral Solvent contenant 3 % d'additif de l'invention, et 300 ppm de benzotriazole. Les solutions, sont chauffées 3 heures à 100°C.

Les résultats obtenus sont rassemblés dans le tableau ci-dessous. Ils sont exprimés par une cotation comprenant un nombre suivi d'une lettre précisant la nuance de la corrosion de la lame de cuivre.

| Additif (3% dans 350NS) | 3h à 100°C |
|---|---|
| II | 1b |
| III | 1b |
| IV | 1b |

Les produits présentant une cotation inférieure à 3, sont particulièrement utiles pour la formulation d'huiles pour engrenages.

**Revendications**

1 - Additifs sulfurés aux lubrifiants, à effet antiusure et extrême-pression caractérisés en ce qu'ils consistent en dihydrocarbylpolysulfures asymétriques renfermant un groupement hydrocarbylpolysulfure R-$S_x$- où R représente un radical aliphatique, aromatique ou hétérocyclique et x varie entre 2 et 10 en position

EP 0 404 607 A2

vicinale d'alcool secondaire ou tertiaire.

2 - Additifs selon la revendication 1 caractérisés en ce que R représente un radical aliphatique en $C_1$ à $C_{18}$ et de préférence en $C_1$ à $C_{14}$, pouvant comporter un ou plusieurs hétéroatomes tel que l'oxygène, le soufre, le phosphore ou l'azote.

3 - Additifs selon la revendication 1 caractérisés en ce que R représente un radical aromatique.

4 - Additifs selon la revendications 3 caractérisés en ce que le radical aromatique est substitué par un ou plusieurs radicaux aliphatiques pouvant renfermer des hétéroatomes.

5 - Additifs selon la revendication 1 caractérisés en ce que R représente un radical hétérocyclique renfermant au moins un hétéroatome choisi parmi le soufre, l'oxygène ou l'azote.

6 - Additifs selon l'une des revendications 1 à 5 caractérisés en ce que la valeur de x varie entre 2 et 6.

7 - Procédé de préparation d'additifs selon les revendications 1 à 6 caractérisé en ce que l'on prépare un sel basique d'un polysulfure $R-S_xM$ par réaction du sel basique d'un mercaptan R-SM où R et x ont la signification précitée et M représente un métal alcalin ou alcalinoterreux ou encore un groupement monovalent, comme $NH_4+$ correspondant à une base minérale MOH avec au moins un équivalent de soufre élémentaire, puis on fait réagir ce polysulfure avec un composé renfermant au moins un groupement époxydique.

8 - Procédé de préparation d'additifs selon les revendications 1 à 6 caractérisé en ce qu'on prépare un thiosulfate organique alpha-hydroxylé par addition d'un thiosulfate alcalin sur un composé renfermant au moins un groupement époxydique, puis on fait réagir le thiosulfate organique alpha-hydroxylé avec le sel basique d'un mercaptan R-SM ou d'un polysulfure organique $R-S_xM$ où R, x et M ont la signification précitée.

9 - Procédé selon la revendication 7 ou 8 caractérisé en ce que les sels basiques de mercaptans sont obtenus par réaction d'un mercaptan avec une base minérale MOH, comme la soude, la potasse ou l'ammoniaque.

10 - Procédé selon l'une des revendications 7 à 9 caractérisé en ce que les sels basiques de polysulfures organiques sont obtenus par réaction des sels basiques de mercaptans avec au moins un équivalent de soufre élémentaire.

11 - Procédé selon la revendication 9 caractérisé en ce que le mercaptan est un mercaptan aliphatique comme le méthylmercaptan, l'éthylmercaptan, le propylmercaptan, le n-butylmercaptan, l'isobutylmercaptan, le ter-bultylmercaptan, le teramylmercaptan, le ternonylmercaptan, le terdodécylmercaptan et le benzylmercaptan.

12 - Procédé selon la revendication 11 caractérisé en ce que le mercaptan aliphatique comporte un ou plusieurs hétéroatomes, comme le mercaptoéthanol, le mercapto-3-propanediol-1,2, l'acide mercapto-acétique ou thioglycolique et l'acide mercapto-propionique.

13 - Procédé selon la revendication 9 caractérisé en ce que le mercaptan est un mercaptan aromatique, comme le phénylmercaptan ou thiophénol, les tolylmercaptans et l'acide ortho-thiobenzoïque ou acide thiosalicylique.

14 - Procédé selon la revendication 9 caractérisé en ce que le mercaptan est un mercaptan hétérocyclique, comme le mercapto-2-imidazole, mercapto-2-méthylimidazole, le mercapto-2-benzimidazole, le mercapto-2-benzoxazole, le mercapto-2-pyridine, le mercapto-4-pyridine, le mercapto-2-thiazole, le mercapto-2-thiazoline, le mercapto-2-méthyl-5-thiadiazole-1, 3,4 et le mercapto-3-méthyl-4-triazole-1,2,4.

15 - Procédé selon la revendication 7 ou 8 caractérisé en ce que le composé renfermant au moins un groupement époxydique a une structure hydrocarbonée acyclique comportant le(s) groupement(s) époxydiques(s) en position terminale, comme l'époxy-1,2-propane, l'époxy-1-2-butane, l'époxy-1,2-héxane, l'époxy-1,2-octane, l'époxy-1,2-dodécane, l'époxy styrène et le diépoxy-1,2,3,4-butane.

16 - Procédé selon la revendication 7 ou 8 caractérisé en ce que le composé renfermant au moins un groupement époxydique a une structure hydrocarbonée acyclique comportant le(s) groupement(s) époxydiques(s) en milieu de chaine, comme les époxydes d'acides gras insaturés, tels que l'acide oléique ou l'oléate de méthyle époxydés, le linoléate de méthyle époxydé ou l'huile de soja époxydée.

17 - Procédé selon la revendication 7 ou 8 caractérisé en ce que le composé renfermant au moins un groupement époxydique a une structure hydrocarbonée cyclique comme l'époxy-1,2-cyclohéxane.

18 - Procédé selon la revendication 7 ou 8 caractérisé en ce que le composé renfermant au moins un groupement époxydique renferme un groupe fonctionnel comme un halogène, un alcool ou un éther en position vicinale par rapport au groupement époxydique comme l'épichlorhydrine, l'épibromhydrine et les éthers alkylglycidiques comme l'éther n-butylglycidique, l'éther éthyl-2-héxylglycidique ou l'éther phénylglycidique.

19 - Composition lubrifiante à effet antiusure et extrêmepression, destinée plus particulièrement à la lubrification des engrenages caractérisée en ce qu'elle renferme une huile lubrifiante minérale ou synthéti-

7

que et 0,5 à 10 % en masse d'un additif selon les revendications 1 à 6.

20 - Composition selon la revendication 19 caractérisée en ce qu'elle renferme d'autre additifs usuels, comme les additifs phosphorés, des antioxydants, des antirouilles, des passivateurs de cuivre, des antimousses et des réducteurs de frottement.

21 - Composition lubrifiante à effet antiusure et extrême-pression, destinée plus particulièrement à des formulations pour travail des métaux, caractérisée en ce qu'elle renferme une huile lubrifiante minérale ou synthétique et 0,1 à 20 % et de préférence 0,5 à 5 % en masse d'un additif selon les revendications 1 à 6.

22 - Composition selon la revendication 21 caractérisée en ce qu'elle renferme d'autre additifs usuels, comme les paraffines chlorées.